# EUROPEAN PATENT APPLICATION

(11) **EP 4 260 854 A1**
(43) Date of publication of application: **18.10.2023**
(21) Application number: 21902682.0
(22) Date of filing: 09.12.2021
(51) Int. Cl.: A61K 31/352, A61K 31/366, A61K 31/365, A61K 31/381, A61P 31/04

(54) **PHARMACEUTICAL APPLICATION OF FUSED-RING PHENOLIC COMPOUND**

(30) Priority: 09.12.2020 CN 202011453853
(71) Applicant: Risen (Suzhou) Pharma Tech Co., Ltd., Suzhou, Jiangsu 215000 (CN)
(72) Inventor: JI, Xiang, Suzhou, Jiangsu 215000 (CN)
(74) Representative: Høiberg P/S
(86) International application number: PCT/CN2021/136707
(87) International publication number: WO 2022/121977

(57) **Abstract**

The present application relates to a kind of fused ring phenolic compound, or a pharmaceutically acceptable salt or ester thereof, in the manufacture of medicaments for treating, inhibiting or preventing bacterial infections. The disclosed fused ring phenolic compounds can inhibit PPK1 and PPK2 and can be used to treat infections or conditions caused by bacteria such as Pseudomonas aeruginosa.

## Description

### Cross Reference

The present application is based on the Chinese patent application numbered 202011453853.2, filed on December 09, 2020, and claims priority from the said application. The whole content of the Chinese patent application is hereby cited as a reference.

### TECHNICAL FIELD

The present invention relates to the use of a kind of fused ring phenolic compound, or a pharmaceutically acceptable salt or ester thereof, in the manufacture of medicaments for treating, inhibiting, or preventing bacterial infections. Specifically, the present invention relates to a PPK inhibitor.

### BACKGROUND OF THE INVENTION

The World Health Organization (WHO) and the developed countries believe that antibiotic-resistant pathogens pose a serious threat to global health, food security, as well as global development. The impact of drug-resistant bacterial pathogens on healthcare systems is enormous. In 2015, the Public Health Agency of Canada disclosed the resulting costs and expenses in the socio-economic sector, productivity, and healthcare, which totaled at least $1 billion. The 2016 "UK Antibiotic Resistance Assessment Report" argues that antibiotic resistance will pose a serious threat to the economy. This assessment report believes that by 2050, antibiotic resistance will lead to a 2% to 3.5% decrease in global GDP, which is equivalent to a direct economic loss of $100 trillion. The report also points out that if the issue of antibiotic resistance is not addressed, 10 million people worldwide will die each year from infections caused by drug-resistant bacteria and pathogens.

In February 2017, the WHO published a list of antibiotic-resistant "priority pathogens," the first kind of it, and announced that *pseudomonas aeruginosa* (a Gram-negative bacterial pathogen) is a "class 1 priority" pathogen for which antibiotics are urgently needed to be developed. Pseudomonas aeruginosa is one of the most important hospital-acquired pathogens and it is easy to infect patients with a compromised immune system (such as patients with cystic fibrosis, cancer, or AIDS) and those who require the use of medical equipment such as respiratory aids and catheters. Pseudomonas aeruginosa can also cause other very serious and fatal infections (such as pneumonia, urinary tract infections, and gastrointestinal infections). Pseudomonas aeruginosa is resistant to many highly potent antibiotics currently in use, such as carbapenems and cephalosporins. In view of the rapid development of drug resistance of bacterial pathogens, research, and development of antibiotic drugs have lagged behind seriously.

Polyphosphate (PolyP) is a linear chain inorganic compound composed of several to hundreds of phosphate groups linked by high-energy phosphoanhydride bonds. It is widely found in all living organisms. Studies have shown that polyP accumulates in bacteria under adverse living conditions, rendering the bacteria highly resistant to environmental stress. Together with its related polyphosphate kinases (PPK) and exopolyphosphatase (PPX), the polyP undergoes stress responses and it is considered a global regulator of the stress and toxicity pathways of various bacterial species. Specifically, PPK is required for quota sensing, biofilm formation, toxin secretion, and virulence in animal infection models of pseudomonas aeruginosa. Inorganic polyphosphate (polyP) is a linear polymer composed of up to 1000 inorganic phosphate monomers polymerized by energy-rich phosphate bonds. In bacteria, polyP is synthesized and consumed by polyphosphate kinases. These enzymes are further divided into two major families, i.e. polyphosphate kinase-1 (PPK1) and polyphosphate kinase-2 (PPK2). The PPK1 enzyme preferentially utilizes ATP as a phosphorylating agent to catalyze the synthesis of polyphosphate. Arthur Kornberg first identified PPK1 as a key virulence determinant of pseudomonas aeruginosa and he proved that quorum sensing, motility, biofilm formation, and virulence of pseudomonas aeruginosa were severely affected after the PPK1 gene was knocked out (Polyphosphate kinase is essential for biofilm development, quorum sensing, and virulence of Pseudomonas aeruginosa. PNAS. 97, 9636-9641). In addition to PPK1, many bacteria possess PPK2 enzyme, which does not have any sequence similarity to PPK1 and whose main function is to use polyP to synthesize GTP rather than synthesizing polyP. PPK2 has been identified in many species, including the major pathogen, as a determinant of virulence and persistence in Pseudomonas aeruginosa, which highlights the overall importance of polyP balance during bacterial infection.

Given the importance of PPK enzymes in bacterial pathogenesis and the lack of homologous enzymes in mammals, PPKs have been dubbed as potential targets for antimicrobial therapy. The current screening efforts have identified several inhibitors of PPK1 and PPK2 enzymes and have resulted in several compounds with relatively weak activity against PPK1 and PPK2 based on bisphosphonates (Burda-Grabowska, M. et al, Bisphosphonic acids and related compounds as inhibitors of nucleotide-and polyphosphate-processing enzymes: A PPK1 and PPK2 case study. Chem Biol Drug Des. 10.1111/cbdd.13439). However, potent compounds (low micromolar or nanomolar activity) against these two kinds of PPKs have not been reported.

### SUMMARY OF THE INVENTION

The technical problem primarily addressed by the present application is to provide potent compounds that target PPK. The applicant has found that specific fused ring phenolic compounds, or pharmaceutically acceptable salts or esters thereof, are capable of effectively inhibiting the production of PPK enzyme, therefore they can be used to prepare the medicaments for the treatment, inhibition or prevention of bacterial infections.

In one aspect, the present invention provides the use of a fused ring phenolic compound in the manufacture of medicaments for treating, inhibiting, or preventing a bacterial infection, wherein, the fused ring phenolic compound comprises one or more of the compounds described in Table 1, or a pharmaceutically acceptable salt or ester thereof.

**Table 1: Fused Ring Phenolic Compound**

| | | | |
|---|---|---|---|
| 1 | 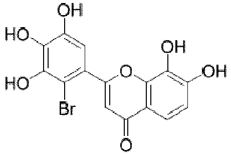 | 6 | 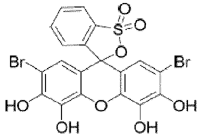 |
| 2 | 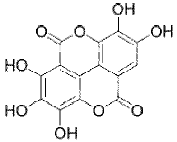 | 7 | 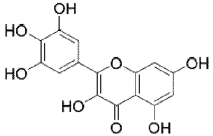 |
| 3 | 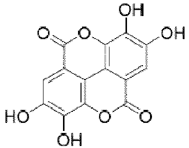 | 8 | 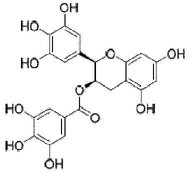 |
| 4 | 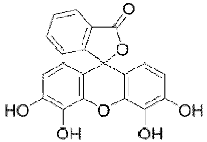 | 9 | 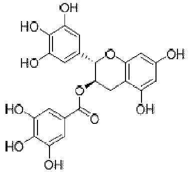 |
| 5 | 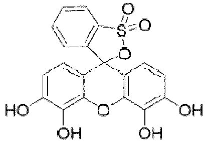 | 10 | 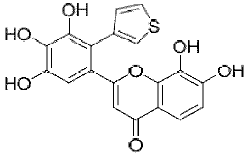 |

Preferably, said bacterial infection comprises bacterial infections caused by a bacterium that produces one or more polyphosphate kinases (PPKs). Specifically, the PPKs include, for example, PPK1, PPK2A, PPK2B, and PPK2C.

In some embodiments, the above bacterial infections include infections caused by *Pseudomonas aeruginosa, Klebsiella pneumoniae, Escherichia coli, Staphylococcus aureus, salmonella, Bacillus subtilis, Acinetobacter baumannii, Enterobacter cloacae, or Enterococcus.* In some specific embodiments, the *Enterococcus* is a vancomycin-resistant *Enterococcus* and the *Staphylococcus aureus* is a methicillin-resistant *Staphylococcus aureus.*

In a particularly preferable embodiment, said bacterial infection comprises a bacterial infection caused by *Pseudomonas aeruginosa.*

Furthermore, the C, H, O and S atoms in the above compounds were independently selected from naturally abundant atoms and isotopically enriched atoms.

Furthermore, the isotopically enriched atoms are selected from the ¹²C, ¹³C and ¹⁴C from carbon, the ¹H, ²H and ³H selected from hydrogen and the ¹⁶O, ¹⁷O and ¹⁸O selected from oxygen as well as the ³²S, ³³S and ³⁴S selected from sulfur.

In addition, the compounds of the present invention may be the listed compounds as well as the pharmaceutically acceptable salts or esters thereof, or may be pharmaceutically acceptable prodrug compounds.

In still another aspect, the present invention provides a medicament for treating, inhibiting or preventing bacterial infections, including one or more of the compounds of the present invention, wherein said bacterial infections include a bacterial infection related to a PPK enzyme-catalyzed reaction.

In a further aspect, the present invention relates to the use of a composition in the manufacture of medicaments for treating, inhibiting or preventing bacterial infections, wherein said compositions include a compound as defined in the present application and at least one pharmaceutically acceptable carrier or diluent.

In some embodiments, the aforementioned medicaments are PPK inhibitors intended for medicaments for treating, inhibiting or preventing bacterial infections caused by bacteria that produce one or more polyphosphate kinases (PPKs), wherein said bacterial infections comprise an infection caused by *Pseudomonas aeruginosa, Klebsiella pneumoniae, Escherichia coli, Staphylococcus aureus, salmonella, Bacillus subtilis, Acinetobacter baumannii, Enterobacter cloacae, or Enterococcus.* As described above, in some specific embodiments, the *Enterococcus* is a vancomycin-resistant *Enterococcus* and the *Staphylococcus aureus* is a methicillin-resistant *Staphylococcus aureus.*

In a particularly preferable embodiment, said bacterial infection comprises a bacterial infection caused by Pseudomonas aeruginosa.

In some embodiments, the aforementioned pharmaceutically acceptable carriers or diluents include creams, emulsions, gels, liposomes, or nanoparticles.

In a further aspect, the present invention provides the use of a kit in the manufacture of medicaments for treating, inhibiting or preventing bacterial infections, wherein said kit comprises a compound or a pharmaceutically acceptable salt or ester of the present application or a composition of the present invention and diluents (e.g., sterile water), buffers and pharmaceutically acceptable excipients.

In yet another aspect, the present invention provides a method of inhibiting the growth of a bacterium *in vitro,* wherein the bacterium thereof is selected from *Pseudomonas aeruginosa, Klebsiella pneumoniae, Escherichia coli, Staphylococcus aureus, Salmonella, Bacillus subtilis, Acinetobacter baumannii, Enterobacter cloacae or Enterococcus,* preferably *Pseudomonas aeruginosa.* The said method comprises: administering a compound described in Table 1 above or a pharmaceutically acceptable salt or ester thereof or a composition of the present application or a kit of the present application to a bacterium at an effective concentration or with an effective amount.

The specific fused ring phenolic compound provided in the present application, or a pharmaceutically acceptable salt or ester thereof, can be used to effectively inhibit the production of PPK enzyme, especially to exert a potent effect against the infections caused by bacteria such as *Pseudomonas aeruginosa* which is regulated by the catalysis of PPK enzyme, therefore they can be used to prepare medicaments for treating, inhibiting or preventing bacterial infections and achieve an excellent therapeutic, inhibitory or prophylactic effect.

### BRIEF DESCRIPTION OF THE DRAWING

Figure 1: IC₅₀ Curves of Compound 4 for Inhibition of Different PPKs (PPK1, PPK2A, PPK2B, and PPK2C).
Figure 2: Effect of Compound 4 on Biofilm Formation in Different Pseudomonas Aeruginosa Strains. A: absorbance. The experimental concentration of compound 4 was 100 µM, and "-" or "+" indicates the absence or presence of compound 4; WT: wild-type strain; Δ *ppk 1* is the PPK1 knocked out strain; Δ *ppk2*A*,* Δ*ppk*2B and Δ*ppk*2C are the strains with PPK2A, PPK2B, PPK2C knocked out; Δ*ppk1,* Δ*ppk*2AΔ*ppk*2B*,* Δ*ppk*2C are the strains with PPK1, PPK2A, PPK2B and PPK2C knocked out.
Figure 3: Effect of Compound **4** on the Survival Rate of Nematodes Infected with Pseudomonas Aeruginosa.
Figure 4: Toxicity Assay of Compound **4** and Compound **B** on Human Embryonic Kidney Cells (HEK Cells): Relative Viability in the Presence of DMSO (Control), Compound **B** (100 µM), and Compound **4** (100 µM).

### DETAILED DESCRIPTION

In order to provide a clear and consistent understanding of the terms used in the specification of the present invention, some definitions are provided below. In addition, all technical and scientific terms used in the present invention have the same meaning as is generally understood by those of ordinary skill in the art of the present invention, unless otherwise specified.

### Definition of Terms

The use of the word "one" may mean "one" when used in conjunction with the term "including" in the claims and/or the specification, but it is also known as the meanings of "one or more", "at least one" and "one or more". Similarly, the word "another" may mean at least a second or many.

The words "including" (and any forms of including, such as "including" and "containing"), "having" (and any forms of having, "having" and "containing"), as used in the present specification and the claims, are inclusive and open-ended and do not exclude additional unlisted elements or processing steps. The term "approximately" or "about" is used to indicate the value including the error resulting from the instruments and methods used to determine the value.

The term "derivative" used in the present invention should be understood to mean another compound similar in structure but distinct in some subtle structure.

In one embodiment, the present invention provides the use of a kind of fused ring phenolic compound, or a pharmaceutically acceptable salt or ester thereof, in the manufacture of medicaments for treating, inhibiting or preventing bacterial infections. And this fused ring phenolic compound is selected but is not limited to the compound listed in Table 1.

A "pharmaceutically acceptable salt" of a compound means a salt of a pharmaceutically acceptable compound. Salts of ideal compounds (basic, acidic or charged functional groups) may retain or improve the biological activity and properties of the parent compound as defined in the present invention and are not biologically indispensable.

In one embodiment, the compounds provided by the present invention may contain an unnatural proportion of isotopic atoms at one or more atoms constituting such compounds. The unnatural proportion of an isotope can be defined from the amount found in nature to the amount made up of 100% of the atoms in question. For example, the compound may incorporate a radioisotope such as tritium (³H) or carbon-14 (¹⁴C) or a non-radioisotope such as deuterium (²H) or carbon-13 (¹³C). Such variations in the isotope may provide additional applications to those described elsewhere in the present application. For example, the isotopic variants of the compounds of the present invention may find additional uses, including, but not limited to, as diagnostic and/or imaging agents, or as cytotoxic/radiotoxic therapeutic agents. In addition, the isotopic variants may have altered pharmacokinetic and pharmacodynamic profiles and this may contribute to enhanced safety, tolerability, or efficacy during the treatment. All isotopic variants of the compounds provided by the present invention, whether or not radioactive, are encompassed by the present invention.

The term "naturally abundant element" or "naturally abundant atom" used in the present invention refers, respectively, to an element or atom that has the most abundant atomic mass in nature. For example, the naturally abundant element of hydrogen is ¹H, the naturally abundant element of sulfur is ³²S, the naturally abundant element of oxygen is ¹⁶O, and the naturally abundant element of carbon is ¹²C. "Non-isotopically enriched" are compounds in which all atoms or elements in the compound are naturally abundant isotopes, meaning that the atomic mass of all atoms or elements is the most abundant in nature.

The fused ring phenolic compound disclosed in the present application, or a pharmaceutically acceptable salt or ester thereof, has a good inhibitory effect on one or both of PPK1 and PPK2, and is used for preparing medicaments for treating, inhibiting or preventing bacterial infections. Among them, bacterial infections include PPK-related bacterial infections, which can be PPK1-related bacterial infections, PPK2-related bacterial infections, or bacterial infections related to both PPK1 and PPK2. The terms "PPK-related bacterial infections" and "bacterial infections mediated by PPK" can be used interchangeably to denote any bacterial infection, disease, disorder, or condition that may benefit from treatment with a PPK inhibitor.

PPK-related bacterial infections involved in the present application encompass those caused by bacteria/pathogens harboring at least one or more of the PPKs. The term "infection" or "bacterial infection" includes the bacteria existing in or on a subject, wherein inhibiting the growth of the bacteria would be beneficial to the object. Therefore, the term "infection" or "bacterial infection" not only refers to the presence of bacteria, but also involves that of unexpected normal bacterial communities. The term "infection" includes infections caused by bacteria. Examples of such bacterial infections include many key bacterial pathogens such as *Pseudomonas aeruginosa, Klebsiella pneumoniae, Acinetobacter baumannii, Francisella tularensis,* and *Campylobacter jejuni,* which all possess at least one PPK1 and PPK2. The genome of *Pseudomonas aeruginosa* not only encodes three PPK2 enzymes, namely PPK2A (PA14_01730), PPK2B (PA14_ 33240) and PPK2C (PA14_19410), but also PPK1 (PA14_69230). Thus, in some embodiments, bacterial infections may be an infection caused by *Pseudomonas aeruginosa* or infection caused by *Klebsiella pneumoniae, Escherichia coli, Staphylococcus aureus, Salmonella, Bacillus subtilis, Acinetobacter baumannii, Enterobacter cloacae, vancomycin-resistant Enterococcus,* or *methicillin-resistant Staphylococcus aureus.*

Wherein, the term "growth" refers to the growth of one or more microorganisms and includes the propagation or population expansion of microorganisms such as bacteria. This term also includes metabolic processes that maintain the continuity of the microorganism, including processes that keep the microorganism alive.

The fused ring phenolic compounds provided by the present invention can be used as PPK inhibitors which can inhibit PPK1 and/or PPK2. The fused ring phenolic compounds described in the present invention have an inhibitory effect on PPK activity and/or anti-inflammatory activity and can be used as a therapeutic agent or prophylactic therapeutic agent when such inhibition is required. Unless otherwise specified, where the use of the compounds of the present invention is described herein, it should be understood that these compounds can be in the form of compositions (i.e., pharmaceutical compositions). As used herein, the terms "PPK inhibitor", "PPK blocker", "polyphosphate kinase inhibitor", "polyphosphate kinase inhibitor" and all the other acceptable terms in relevant fields can be interchangeably used to refer to compounds that can directly or indirectly inhibit PPK receptors in *in vitro* testing, in vivo models, and/or other tests demonstrating PPK inhibition and potential therapeutic or preventive efficacy. This term also refers to a compound that exhibits at least some therapeutic or prophylactic benefit in a human subject.

The present invention also provides a bacteriostatic agent/medicament, including one or more of the above compounds, for treating, inhibiting or preventing bacterial infections, especially those associated with PPK, wherein the bacterial infections thereof include infections caused by *Pseudomonas aeruginosa, Klebsiella pneumoniae, Escherichia coli, Staphylococcus aureus, salmonella, Acinetobacter baumannii, Bacillus subtilis, Enterobacter cloacae* or *Enterococcus.* The term "antibacterial agent" refers to any substance, compound, or their compositions that can inhibit, reduce, or prevent bacterial growth, inhibit or reduce the ability of bacteria to cause infection in the object, or inhibit or reduce the ability of bacteria to reproduce or maintain infection in the environment, or reduce the infectivity or toxicity of bacteria.

The present invention provides pharmaceutical compositions including a compound disclosed in the present invention, or a pharmaceutically acceptable salt or ester thereof, and a pharmaceutically acceptable carrier. In some embodiments, a pharmaceutical composition is provided that includes at least one of compounds 1 to 10, or a pharmaceutically acceptable salt or ester thereof, and a pharmaceutically acceptable carrier.

In other embodiments, the present invention provides a method for treating and/or preventing immune-related diseases, disorders, and symptoms, diseases with inflammatory components, and disorders related to the above, using at least one PPK inhibitor compound or composition provided by the present invention.

Other diseases, disorders, and conditions that can be completely or partially treated or prevented by inhibiting PPK activity are also candidate indications for the PPK inhibitor compounds and compositions provided by the present invention.

The term "treatment" refers to the initiation of actions (such as the administration of PPK inhibitors or drug compositions containing PPK inhibitors) after the diagnosis and observation of a disease, disorder, or condition or its symptoms, in order to temporarily or permanently eliminate, alleviate, suppress, slow down, or improve at least one potential cause of the disease, disorder, or condition that torments the subject, or symptoms related to the disease, disorder, or condition that torments the subject. Treatment therefore includes inhibition (for example, preventing or mitigating the progression or further progression of a disease, disorder or condition or its associated clinical symptoms) of an active disease. Specifically, the term "treatment" used herein is used to specifically refer to the administration of a therapeutic substance comprising a compound or composition according to the present invention to a patient who has already contracted an infection. The term 'treatment' also relates to the use of a compound or combination according to the present invention, optionally in conjunction with one or more antibacterial agents, to reduce or alleviate bacterial infections or one or more of the symptoms associated with bacterial infections; or slow down the development of bacterial infections or one or more symptoms related to bacterial infections; or reduce the severity of bacterial infections or the severity of one or more symptoms related to bacterial infections; or inhibit the clinical manifestations of bacterial infections; or inhibit the manifestation of adverse symptoms of bacterial infection.

The term 'prevention' refers to taking action in a certain way (such as before the onset of a disease, disorder, disease or its symptoms) (such as administering PPK inhibitors or drug compositions containing them), in order to temporarily or permanently prevent, inhibit, suppress, or reduce the risk of a subject suffering from a disease, disorder, condition, etc. (such as determined by the lack of clinical symptoms) or delay the onset of specific diseases, disorders, or conditions to which a subject is predisposed to. In some cases, the term also refers to slowing down the progression of a disease, disorder, or condition or inhibiting its progression to a harmful or other undesirable status. Specifically, the term "prevention" used herein refers to the application of compounds or compositions according to the present invention to prevent bacterial infections or the occurrence of related infections and/or diseases. The term "prophylaxis" also encompasses the prevention of at least one bacterial infection by administration of a compound or composition according to the present invention to a patient susceptible to or at risk of being infected by a bacterium.

In some embodiments, the present invention further provides the use of the PPK inhibitor compounds and compositions described herein in combination with one or more additional agents. One or more additional agents may have some PPK-modulating activity and/or they may act through a different mechanism of action. In some embodiments, such agents comprise radiation (e.g., local radiation therapy or total body radiation therapy) and/or other forms of treatment that are non-pharmacological in nature. When a combinational therapy is used, the PPK inhibitor and one additional agent may be used in the form of a single composition or multiple compositions, and the treatments may be administered simultaneously, sequentially, or using some other regimen. For example, in some embodiments, embodiments are provided in which a radiation phase is followed by a chemotherapy phase. Combinational therapy may have an additive effect or a synergistic effect.

Pharmaceutical compositions containing an active ingredient (e.g., a PPK inhibitor) may be in a form suitable for oral use, such as tablets, capsules, lozenges, sugar lozenges, aqueous or oily suspensions, dispersible powders or granules, emulsions, hard or soft capsules, or syrups, solutions, microbeads or elixirs. Pharmaceutical compositions for oral use may be prepared according to any process known in the art for the manufacture of pharmaceutical compositions, and such compositions may contain one or more agents, such as sweeteners, flavors, coloring agents, and preservatives to provide a pharmaceutically acceptable preparation. Tablets, capsules, etc. typically contain active ingredients mixed with non-toxic pharmaceutically acceptable carriers or excipients suitable for manufacturing tablets. These carriers or excipients may be, for example, diluents such as calcium carbonate, sodium carbonate, lactose, calcium phosphate or sodium phosphate; granulating and disintegrating agents, such as corn starch or alginic acid; binders such as starch, gelatin or gum arabic and lubricants such as magnesium stearate, stearic acid or talc.

In some embodiments, the composition is an injectable preparation. In other embodiments, the composition is formulated for oral administration to a subject.

In some embodiments, the pharmaceutical composition is contained in a single-use container (e.g., a single use vial, ampoule, syringe, or autoinjector), while in other embodiments, it is contained in a multi-use container (e.g., a multi-use vial).

The preparation may also include a carrier to protect the composition from rapid degradation or disappearance from the body, such as controlled release preparations, including liposomes, hydrogels, and microencapsulated delivery systems. For example, a controlled released material, such as glycerol monostearate or glycerol stearate alone, or in combination with a wax, may be used. Any drug delivery device can be used to deliver PPK inhibitors, including implants (e.g., implantable pumps) and catheter systems, slow syringe pumps and devices. All of these are well known to those skilled in the art. The pharmaceutical composition may also be in the form of a sterile injectable aqueous or oily suspension. The suspension may be prepared according to known technologies using those suitable dispersants or wetting agents and suspending agents mentioned herein. The sterile injectable preparation may also be a sterile injectable solution or suspension in a non-toxic parenterally acceptable diluent or solvent, such as the solution in 1,3-butanediol. Acceptable diluents, solvents, and dispersion media that may be used include water, Ringer's solution, isotonic sodium chloride solution, Cremophor ELTM (BASF, Parsippany, NJ) or phosphate buffered saline (PBS), ethanol, polyols such as glycerine, propylene glycol, and liquid polyethylene glycols, and suitable mixtures thereof. In addition, sterile fixation oils are often used as solvents or suspension media. For this purpose, any mild fixed oil can be used, including synthetic monoglyceride or diglyceride. Moreover, fatty acids such as oleic acid can be used to prepare injections. Prolonged absorption of specific injectable formulations may be achieved by including agents that can delay absorption (e.g., aluminum monostearate or gelatin).

The PPK inhibitor compounds and compositions provided by the present invention may be administered to a subject in any appropriate manner known in the art. Suitable routes of administration include, but are not limited to, oral administration; parenteral routes, such as intramuscular, intravenous, subcutaneous (e.g., injection or implantation), intraperitoneal, intracisternal, intra-articular, intracerebral (intraparenchymal and intraventricular), nasal, vaginal, sublingual, intraocular, rectal, topical (e.g., transdermal) and oral and inhalation. Depot injection, generally by subcutaneous or intramuscular administration, can also be used to release the PPK inhibitors disclosed herein over a defined period of time.

The present invention also provides kits comprising PPK inhibitor compounds or compositions. The kits are generally in the form of a physical structure containing the various components and can be used, for example, to carry out the methods provided herein. For example, a kit may include one or more PPK inhibitors disclosed in the present invention (e.g., supplied in a sterile container), which may be in the form of a pharmaceutical composition suitable for administration to a subject. The PPK inhibitor may be provided in ready-to-use (e.g., tablets or capsules) form or in a form that requires reconstitution or dilution (e.g., powder), for example, prior to administration. When the PPK inhibitors are in a form that requires to be reconstituted or diluted, the kit may also include diluents (e.g., sterile water), buffers, pharmaceutically acceptable excipients, etc. packaged with the PPK inhibitor or separately packaged. When a combinational therapy is employed, the kits may contain several therapeutic agents independently, or they may have been combined in a single kit. Each component in the kit may be enclosed within a separate container, and all of the various containers may be supplied in a single package. The kits of the present invention may be designed for the conditions (e.g., refrigeration or frozen) required to properly maintain the components contained therein.

In order to better understand the present invention and demonstrate more clearly how to implement it, the features of the embodiments as per the present invention are elaborated through examples and with reference to the accompanying drawings.

### Detailed Description of the Invention

It will be easier to understand the present invention by referring to the following embodiments, which are used to illustrate the present invention and should not be interpreted as limiting the scope of the present invention in any way.

Unless otherwise defined or expressly specified in the context, all technical and scientific terms used herein have the same meaning as generally understood by ordinary technicians in the field to which the invention belongs. It should be understood that any methods and materials similar to or equivalent to those described herein may be used in practice or testing of the present invention.

### Materials

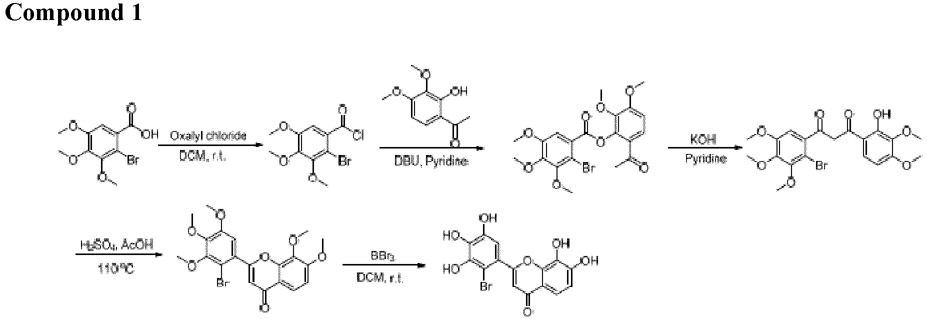

Compound 1 was synthesized using 2-bromo-3, 4, 5-trimethoxybenzoic acid (2.67 g, 9.17 mmol) as the starting material.

¹H NMR (DMSO-*d*₆, 500MHz) δ ppm 6.26 (s, 1H), 6.70(s, 1H), 6.94 (d, *J*=8.5Hz, 1H), 7.38 (d, *J* =8.5Hz, 1H).¹³C NMR (DMSO-*d*₆, 125MHz) δ ppm 100.05, 109.13, 110.81, 114.03, 114.97, 116 .87, 123.97, 133.32, 136.51, 144.30, 145.00, 146.98, 150.27, 163.77, 176.67; m/z (ESI⁺):380.8, 3 82.8.

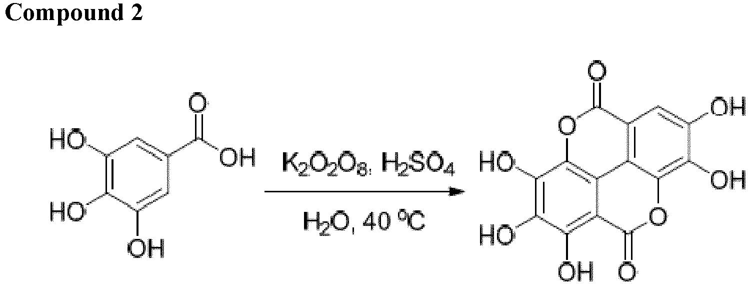

The concentrated suspension of gallic acid (10 g, 5.9 mmol) was added with H₂SO₄ (80mL) and H₂O (33mL), and cooled to -50°C, and then K₂S₂O₈ (20 g, 7.4 mmol) was added. The precipitate was filtered and recrystallized to obtain **compound 2** (2.0 g, 10.4%) by stirring at 4°C for 16h.

¹H NMR (DMSO-*d*₆, 500MHz) δ ppm 7.49(s, 1H);¹³C NMR (DMSO-*d*₆, 125MHz) δ ppm 93.98, 108.71, 109.51, 110.75, 113.09, 130.92, 135.19, 136.29, 140.02, 142.26, 146.90, 148.68, 159.70, 162.91; m/z(ESI-):316.8.

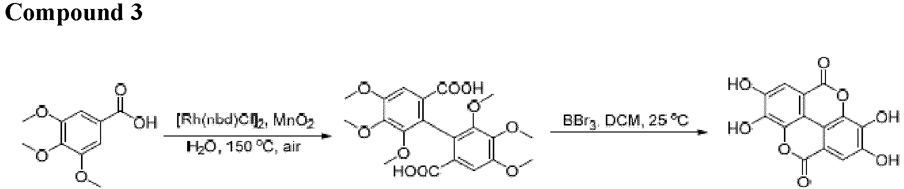

3, 4, 5-trimethoxybenzoic acid (1 g, 4.7 mmol), [Rh (NBD) Cl]₂ (13.8 mg, 0.03 mmol, 0.6 mol%) and MnO₂ (1.23 g, 14.1 mmol, 3 equiv) were dissolved in distilled water (5 mL) and stirred at 150°C for 72 h in a sealed tube. The reaction mixture was cooled to room temperature, acidified to pH < 3 with 2M HCl, and concentrated. The mixture was purified by column chromatography (MeOH/DCM=1/20-1/5) to afford 4, 4', 5, 5', 6, 6'-hexamethylmethoxy-[1,1'-biphenyl]-2,2'-dicarboxylic acid (702 mg, 35.3%). At 0°C and under a nitrogen atmosphere, BBr3 (2 mL, 21.5 mmol) was added dropwise to a solution of 4, 4', 5, 5', 6, 6'-hexamethoxy-[1,1'-biphenyl]-2,2'-dicarboxylic acid (108 mg, 0.25 mmol) in DCM (25 mL). The temperature of the mixture was slowly raised to room temperature and stirred at room temperature for an additional 40 h. The mixture was then slowly poured into ice and the solids were collected by filtration and washed with cold methanol. The resulting solid was dried under vacuum to obtain compound 3 (75 mg, 99.3%).

¹H NMR (DMSO-*d*₆, 500Hz), δ ppm 7.46 (s, 2H), 10.55 (s, 2H), 10.80(s, 2H); ¹³C NMR(DMSO-d₆, 125Hz), δ ppm107.71, 110.22, 112.34, 136.53, 139.47, 148.11, 159.20; m/z (ESI-)301.1.

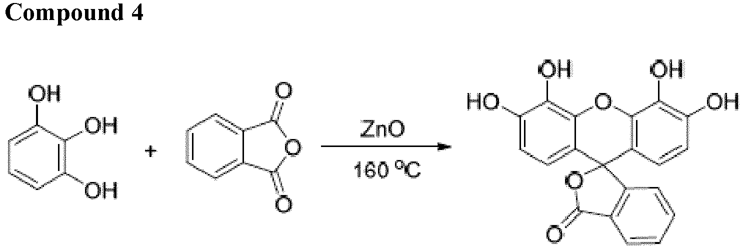

ZnO (81.4 mg, 1.0 mmol) was added to a mixture of phthalic anhydride (1.5 g, 10 mmol) and pyrogallol (2.5 g, 20 mmol), heated to 160°C and kept at this temperature for 2 h, and then cooled to room temperature. The mixture was dissolved in 1N NaOH (10 mL) and filtered. The filtrate was acidified to pH 7 and the solid was collected by filtration. The resulting solid was recrystallized with MeOH and dried to obtain compound 4 (2.2 g, 61.1%).

¹H NMR (CD3OD, 500 MHz) δ ppm 6.09 (d, J=8.0 Hz, 2H), 6.56 (d, J=8.5Hz, 2H), 7.22 (d, J=7. 5Hz, 1H), 7.68 (t, J=7.0Hz, 1H), 7.75 (t, J=7.5Hz, 1H), 7.97 (d, J=7.5Hz, 1H);¹³C NMR (CD3OD, 125MHz) δ ppm 111.12, 111.62, 117.52, 124.14, 124.43, 126.82, 129.58, 132.63, 134.89, 140.7 0, 146.89, 170.10; m/z (ESI⁺):365.3.

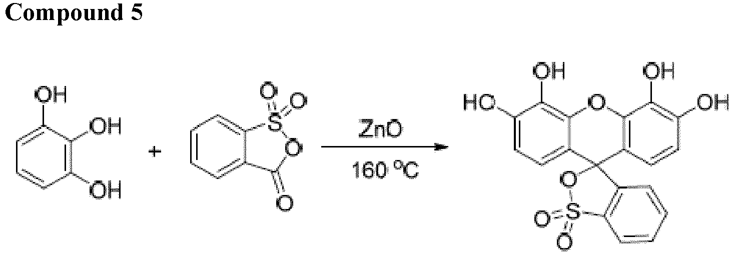

Compound **5** was obtained using the method for compound **4** and using 3H-benzo [c] [1,2] oxathi ol-3-1,1-dioxide as the starting material.

¹H NMR (CD3OD, 500MHz) δ ppm 7.17-7.22 (m, 4H), 7.41 (d, J=7.5Hz, 1H), 7.74 (t, J=8.0Hz, 1H), 7.83 (t, J=8.0Hz, 1H), 8.24 (d, J=7.5Hz, 1H). 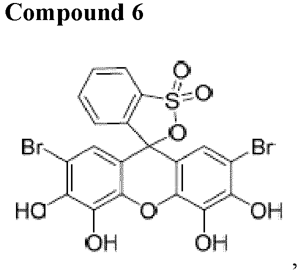 Compound 6 is a commercial product purchased from Shanghai Yuanye Bio-Technology Co., Ltd. 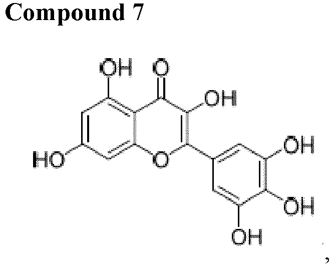 Compound 7 is a commercial product purchased from Shanghai Yuanye Bio-Technology Co., Ltd. 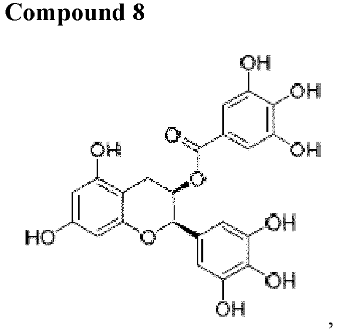 Compound **8** is a commercial product purchased from J&K Scientific. 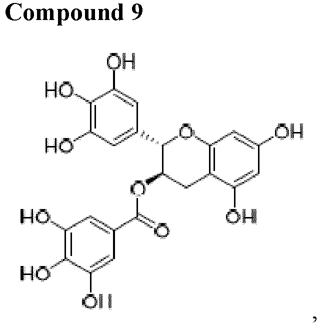 Compound 9 is a commercial product purchased from Aldrich.

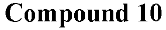

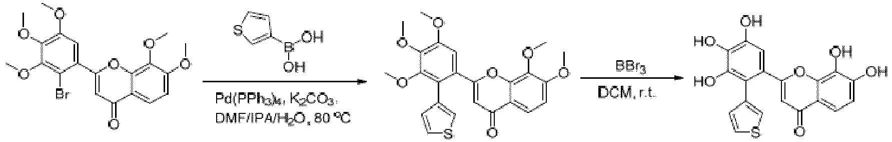

As shown above, Compound **10** is synthesized using thiophen-3-yl boronic acid as the starting material.

¹H NMR (DMSO-d6,500 MHz) δ ppm 5.61 (s, 1H), 6.79 (s, 1H), 6.88 (d, J=8.5Hz, 1H), 6.94 (dd, J1=4.SHz, J2=1Hz, 1H), 7.21-7.22 (m, 1H), 7.25 (d, J=8.5Hz, 1H), 7.40-7.42 (m, 1H); ¹³C NMR (DMSO-d6, 125MHz) δ ppm108.03, 110.36, 113.97, 114.78, 115.62, 116.70, 123.14, 123.89, 12 4.63, 130.12, 133.29, 135.77, 136.21, 144.65, 145.04, 146.90, 149.99, 164.57, 176.32; m/z (ESI⁺): 384.8.

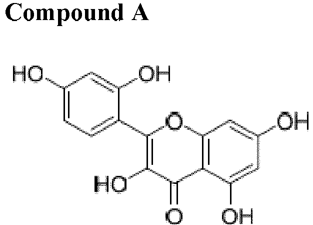

, Compound **A** is a commercial product purchased from J&K Scientific.

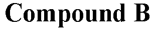

This compound can also be purchased from commercial sources and the compounds used in the present application are synthesized through the following reactions:

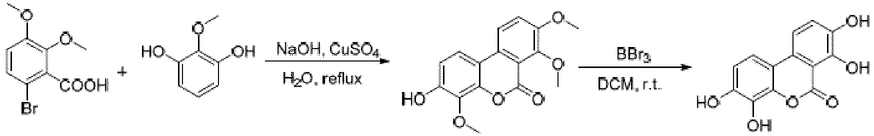

This compound was synthesized using 2-methoxybenzene-1,3-diol as the starting material.

¹H NMR (DMSO-d6, 500MHz) δ ppm 6.87 (d, J=8.7Hz, 1H), 7.36 (d, J=8.5Hz, 1H), 7.51 (d, J=8 .7Hz, 1H), 7.57 (d, J=8.6Hz, 1H), 9.31 (s, 1H), 9.70 (s, 1H), 9.80 (s, 1H), 11.22 (s, 1H). ¹³C NMR (DMSO-d6, 125MHz) δ ppm 105.45, 111.48, 112.65, 113.09, 113.41, 124.73, 127.20, 133.36, 13 9.96, 144.54, 147.25, 149.59, 165.78; m/z(ESI-)258.8.

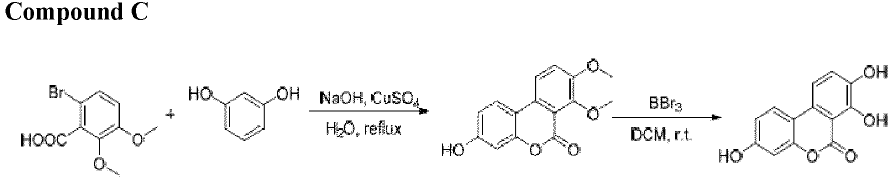

This compound was synthesized using 6-bromo-2,3-dimethoxybenzoic acid as the starting material.

¹H NMR (DMSO-d6, 500MHz) δ ppm 3.82 (s, 3H), 3.93 (s, 3H), 6.69 (d, J=2.5Hz, 1H), 6.81 (dd, J1=2.SHz, J2=9.0Hz, 1H), 7.66 (d, J=9.0Hz, 1H), 8.01 (d, J=9.0Hz, 1H), 8.08 (d, J=9.0Hz, 1H). ¹³C NMR (DMSO-d6, 125MHz) δ ppm 56.76, 61.11, 102.77, 109.79, 113.35, 113.95, 118.01, 121 .01, 124.81, 129.35, 150.82, 151.76, 152.56, 157.30, 159.42; m/z(ESI⁺)272.8.

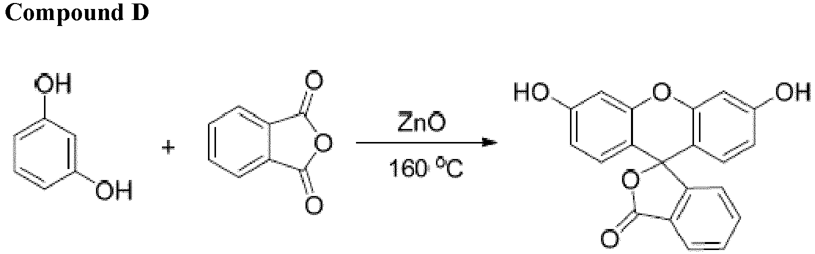

This compound was synthesized using resorcinol as the starting material.

¹H NMR (DMSO-d6, 500MHz) δppm 6.56-6.58 (m, 4H), 6.71-6.72 (m, 2H), 7.28-7.30 (m, 1H), 7.70-7.74 (m, 1H), 7.78 (m, 1H), 7.80-7.82 (m, 1H), 8.00-8.02 (m, 1H).

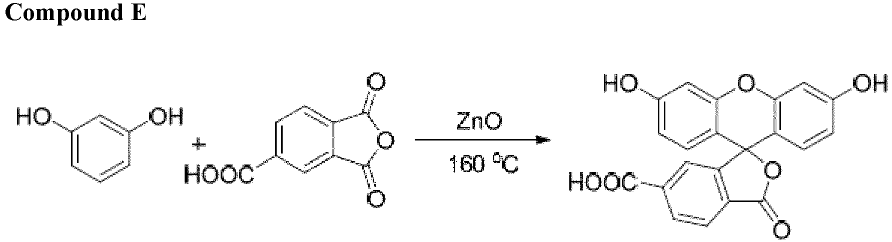

This compound was synthesized using 1,3-dioxo-1,3-dihydroisobenzofuran-5-carboxylic acid as the starting material.

¹H NMR (CD3OD, 500MHz) δ ppm 7.17 (dd, J1=2.0Hz, J2=9.0Hz, 2H), 7.34 (d, J=2.0Hz, 2H), 7.47 (d, J=9.0Hz, 2H), 8.04 (s, 1H), 8.45-8.47 (m, 2H). 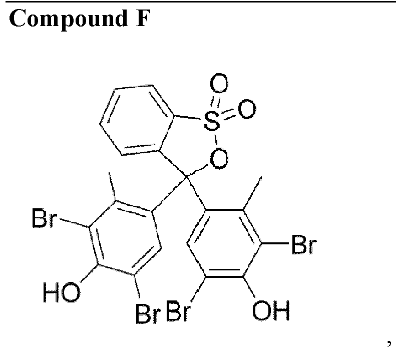 Compound **F** is a commercial product purchased from Aldrich. 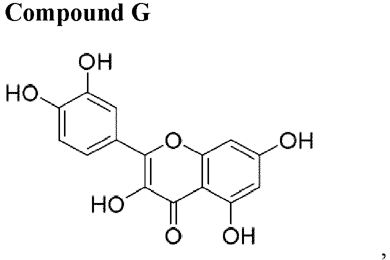 Compound **G** is a commercial product purchased from Aldrich. 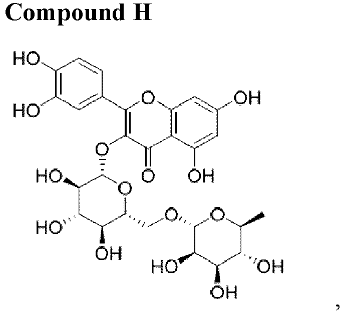 Compound H is a commercial product purchased from TCI.

### Biological Assays

### 1. Antibacterial Assay

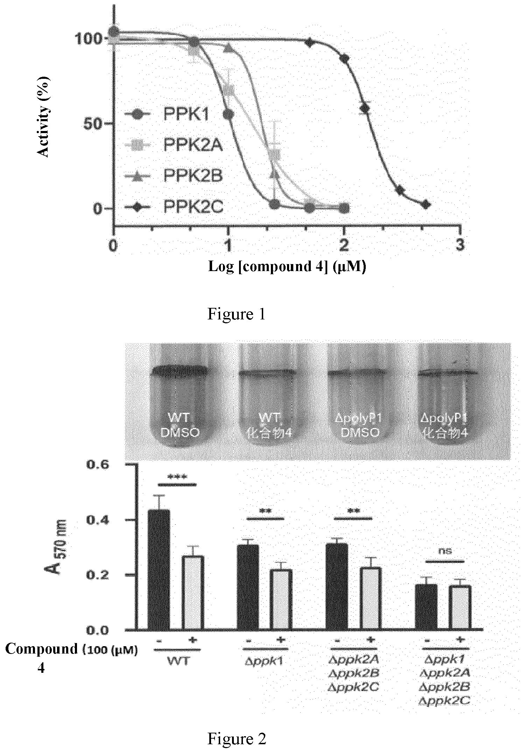

In order to evaluate the inhibitory effect of the compounds on PPKs, the PPK1, PPK2A, PPK2B and PPK2C were used respectively to test the above compounds using a kit described in an article (Liang Peng et al. & Ning Sun (2020) Discovery and antibacterial study of potential PPK1 inhibitors against uropathogenic E. coli, Journal of Enzyme Inhibition and Medicinal Chemistry, 35:1, 1224-1232). The test results obtained from the PPK inhibitor screening assays for the compounds are shown in Table 1 and Table 2. The data in Table 1 show that the compounds of the present invention have good biological activity. Among them, the test results of the inhibitory effect of compound **4** on the PPKs are shown in Figure 1.

**Table 1. IC₅₀ Values for PPK Inhibition by the Above Compounds**

| Compound No. | IC₅₀ /µM | IC₅₀ /µM | Compound No. |
|---|---|---|---|
| 1 | 6 | Inactive | A |
| 2 | 8 | 150 | B |
| 3 | 9 | >100 | C |
| 4 | 17 | >200 | D |
| 5 | 26.3 | >200 | E |
| 6 | 7.59 | Inactive | F |
| 7 | 39 | 177 | G |
| 8 | 16.6 | Inactive | H |
| 9 | 31 | | |
| 10 | 36 | | |

**Table 2. IC₅₀ Values for PPK1 and PPK2 Inhibition by Some Compounds**

| Compound No. | PPK1 | PPK2A | PPK2B | PPK2C |
|---|---|---|---|---|
| 2 | 20 | N/A | 59 | N/A |
| 3 | 20 | 50 | 48 | N/A |
| 4 | 17 | 16 | 20 | 165 |
| 5 | 26 | 24 | 31 | 273 |
| B | 120 | N/A | N/A | N/A |

### 2. Biofilm Testing

Pseudomonas aeruginosa can form biofilm in culture medium. Four groups of LB liquid media were set respectively, among them, group A was inoculated with wild-type Pseudomonas aeruginosa (WT); group B was inoculated with PPK1 knocked out Pseudomonas aeruginosa (Δ ppk1); group C was inoculated with PPK2A, PPK2B and PPK2C knocked out Pseudomonas aeruginosa (Δ ppk 2A, Δ ppk 2B, Δ ppk 2C); group D was inoculated with PPK1 and PPK2A, PPK2B and PPK2C knocked out Pseudomonas aeruginosa (Δ ppk1, Δ ppk 2A Δ ppk 2B, Δ ppk 2C). After the four groups were infected with Pseudomonas aeruginosa, each group was further divided into two groups, with one group added with DMSO (-) as the control, the other group added with 100 µm compound 4 (+). After the bacteria were cultured at 37°C for 24 h, a layer of biofilm was formed in the culture medium. And this biofilm formed in the culture medium of groups A, B, C and D was analyzed using 570 nm visible light. The results showed that Compound 4 had a good inhibitory effect on wild-type Pseudomonas aeruginosa and could reduce the formation of biofilm; moreover, this compound not only inhibited PPK1, but also exhibited a good inhibitory effect on PPK2. The specific results are shown in Figure 2.

### 3. Nematode Survival Test

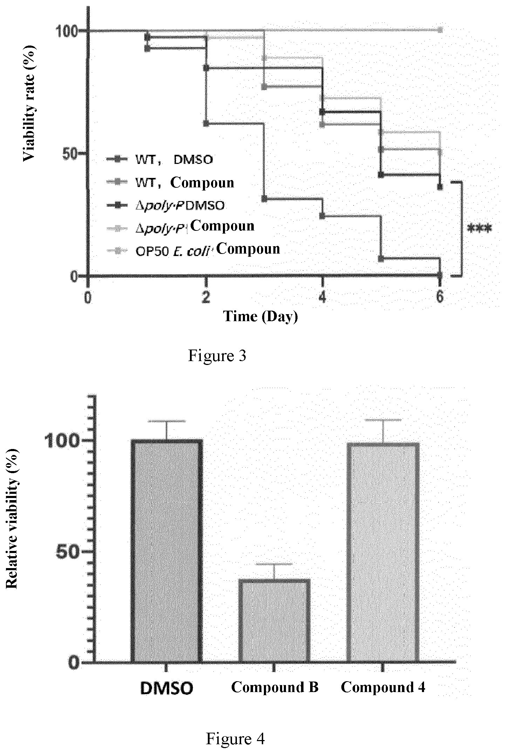

Caenorhabditis elegans were divided into five groups A, B, C, D, E, and then subjected to modeling respectively. Caenorhabditis elegans in group A and B were infected with wild-type (WT) Pseudomonas aeruginosa. Group A served as the DMSO control group and group B as compound 4 treatment group. Caenorhabditis elegans in groups C and D were infected with Pseudomonas aeruginosa which was knocked out of all PPK (Δ*polyP,* Δ*ppk1*Δ*ppk2A*Δ*ppk2B*Δ *ppk 2C*)*,* where group C was DMSO control group and group D served as compound 4 treatment group; and group E was infected with *Escherichia coli* (OP50E. *Coli*)*.* After modeling, Caenorhabditis elegans in groups A and C were continuously treated with DMSO, and those in groups B, D and E were continuously treated with compound 4. The environment and conditions were consistent across the five groups. Six days later, the survival rate of nematodes in five groups was calculated. The test results are shown in Figure 3. The results showed that after infected by wild-type Pseudomonas aeruginosa, nematodes were prone to death, and the mortality was significantly reduced after treatment with compound 4, and the therapeutic efficacy of compound 4 was similar to that of Pseudomonas aeruginosa knocked out of PPK.

### 4. HEK Cytotoxicity Test

The cytotoxicity of the compounds was assessed in cultured human embryonic kidney cells (HEK239T) using the Trypan blue staining cell viability assay. The blank solvent DMSO, compound **B,** and compound **4** were tested, respectively. The test results are shown in Figure 4. The results showed that compound **4** had no cytotoxicity at concentrations up to 100 µM (relative viability rate was close to 100%), while compound **B** caused a large number of cell death (relative viability rate was approximately 35%).

The fused ring phenol compounds disclosed in the present application are not only highly active but also safe (minimal or no cytotoxicity); and they have very good inhibitory effect on PPK1 and PPK2. Therefore, it is feasible to use such compounds to prepare drugs for the treatment, inhibition or prevention of PPK-related bacterial infections.

Although the present invention has been described in detail with reference to its embodiments, these embodiments are provided to illustrate rather than limit the scope of the present invention. Other embodiments obtainable in accordance with the principles of the present invention fall within the scope defined in the claims of the present invention.

## Claims

1. The said use of a fused ring phenolic compound for the manufacture of a medicament for treating, inhibiting or preventing bacterial infections. The said compounds are selected from one or more of the following: 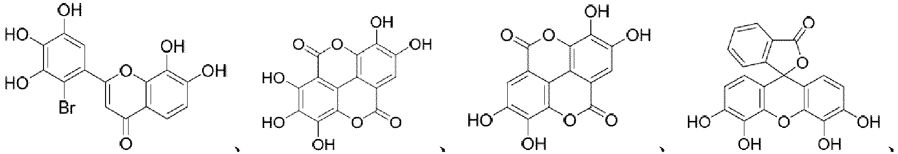 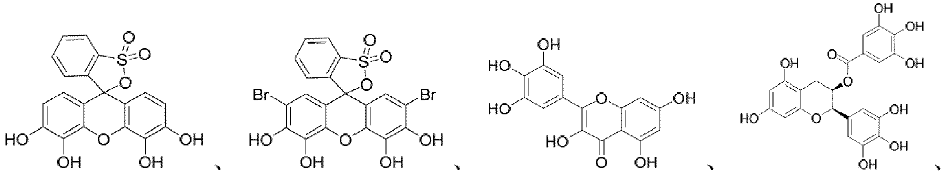 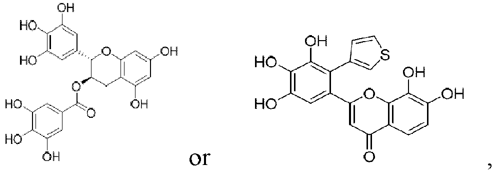 or a pharmaceutically acceptable salt or ester thereof.

2. The said use of claim 1, wherein the bacterial infections thereof include a bacterial infection caused by a bacterium producing one or more polyphosphate kinases (PPKs), including, for example, PPK1 and PPK2A, PPK2B, PPK2C.

3. The said use of claim 1 or 2, wherein the bacterial infections include infections caused by *Pseudomonas aeruginosa, Klebsiella pneumoniae, Escherichia coli, Staphylococcus aureus, Salmonella, Bacillus subtilis, Acinetobacter baumannii, Enterobacteriaceae cloacae* or *Enterococcus.*

4. The said use of claim 1, wherein the C, H, O, and S atoms in the compound are independently selected from atoms of natural abundance and atoms enriched in isotopic abundance.

5. The said use of claim 4, wherein the said isotopically enriched atoms are selected from the ¹²C, ¹³C and ¹⁴C from carbon, the ¹H, ²H and ³H selected from hydrogen and the ¹⁶ O, ¹⁷O and ¹⁸O selected from oxygen as well as the ³²S, ³³S and ³⁴S selected from sulfur.

6. A medicament for treating, inhibiting or preventing bacterial infections, comprising one or more of the compounds as defined in any one of claims 1 to 5, wherein the bacterial infections thereof comprise a PPK-related bacterial infection.

7. The said use of a composition in the manufacture of medicaments for treating, inhibiting or preventing bacterial infections, wherein the said compositions comprising one or more of the compounds as defined in any one of claims 1 to 5 and at least one pharmaceutically acceptable carrier or diluent.

8. The said use of claim 7, wherein said bacterial infection comprises bacterial infections caused by a bacterium that produces one or more polyphosphate kinases (PPKs).

9. The said use of claim 7 or 8, wherein the bacterial infection includes infection caused by *Pseudomonas aeruginosa, Klebsiella pneumoniae, Escherichia coli, Staphylococcus aureus, Salmonella, Acinetobacter baumannii, Bacillus subtilis, Enterobacteriaceae cloacae* or *Enterococcus.*

10. The said use of claim 7, wherein the medicinal carriers or diluents include cream, emulsion, gel, liposome or nanoparticles.

11. The said use of any one of claims 7-10, wherein said composition is suitable for oral administration or can be used for injection administration.

12. The said use of a reagent kit in the preparation of medicaments for treating, inhibiting, or preventing bacterial infections, wherein the reagent kit thereof comprises a compound or pharmaceutically acceptable salt or ester as defined in any one of claims 1 to 5, or a composition as defined in any one of claims 7-11; and diluents (such as sterile water), buffers, and pharmaceutically acceptable excipients.

13. The said use of a compound or combination or reagent kit in inhibiting the production of PPK enzymes, wherein the PPK thereof comprises, for example, PPK1 and PPK2A, PPK2B, PPK2C, and the compound thereof is a compound or pharmaceutically acceptable salt or ester defined in any one of claims 1-5; and said composition is defined in any one of claims 7-11 and the reagent kit is defined of claim 12.
